# EUROPEAN PATENT APPLICATION

(11) **EP 4 292 471 A1**
(43) Date of publication of application: **20.12.2023**
(21) Application number: 22178615.5
(22) Date of filing: 13.06.2022
(51) Int. Cl.: A46B 15/00, A61C 19/06, A61N 1/44, A61C 17/00

(54) **ELECTROLYSIS DEVICE AND METHOD OF OPERATING THE SAME**

(71) Applicant: Koninklijke Philips N.V., 5656 AG Eindhoven (NL)
(72) Inventor: PANDE, Nakul, Eindhoven (NL); GERHARDT, Lutz Christian, Eindhoven (NL)
(74) Representative: Philips Intellectual Property & Standards

(57) **Abstract**

Provided is an oral care device (100) for dental tartar removal. The oral care device comprises a control arrangement (102) configured to provide an electrical signal, for example a timevarying electrical signal. The oral care device also comprises a pair of electrodes (104A, 104B) for contacting an aqueous solution. The pair of electrodes are each connected to the control arrangement and configured to, responsive to said electrical signal, generate hydrogen ions from water in the aqueous solution to deliver within a subject's oral cavity, in particular to the subject's teeth. A variation of the electrical signal controls said generation of hydrogen ions. The thus generated hydrogen ions can lower a pH local to the subject's teeth and thereby assist to dissolve tartar thereon. The variation, for example time-variation, of the electrical signal may provide control over the generation of hydrogen ions which contrasts with, for instance, an uncontrolled generation of hydrogen ions via a non-varying voltage across the pair of electrodes provided by a direct current source. Further provided is a method of operating such an oral care device.

## Description

### FIELD OF THE INVENTION

This invention relates to a device, in particular an oral care device, comprising a pair of electrodes for electrolysing water to generate hydrogen ions. The invention further relates to a method of operating the device.

### BACKGROUND OF THE INVENTION

Build-up of calcified deposits in the body can be problematic. An example of a calcified deposit is dental tartar.

Dental tartar, or early dental calculus, is a risk factor for gingivitis and periodontitis. Dental tartar has different calcium phosphate crystal phases which differ in solubility. The poor aqueous solubility of some of the calcium phosphate crystal phases can make the dental tartar difficult to remove.

At present, dental tartar tends to be treated/removed only by dental practitioners and oral hygienists using mechanical or ultrasound scalers.

It would be desirable to provide further dental tartar treatment/removal options, particularly ones which can be used in a patient's own home.

### SUMMARY OF THE INVENTION

The invention is defined by the independent claims. The dependent claims define advantageous embodiments.

According to examples in accordance with an aspect of the invention there is provided an oral care device comprising: a control arrangement configured to provide an electrical signal; and a pair of electrodes for contacting an aqueous solution, the pair of electrodes being connected to the control arrangement, the electrical signal and the pair of electrodes being configured such that at least one of the electrodes, responsive to said electrical signal, generates hydrogen ions from water in the aqueous solution to deliver within a subject's oral cavity, a variation of the electrical signal controlling said generation of hydrogen ions.

The hydrogen ions may assist to lower a local pH proximal to the subject's teeth to assist dissolution of dental tartar. The variation, for example time-variation, of the electrical signal may provide control over the generation of hydrogen ions which contrasts with, for instance, an uncontrolled generation of hydrogen ions via a non-varying voltage across the pair of electrodes provided by a continuously applied direct current source. This may permit the local pH to which the subject's teeth are exposed to be more closely controlled, thereby facilitating at-home use of the oral care device.

It is noted that the term "electrical signal" as used herein may refer to a voltage or a current signal.

In some embodiments, the variation of the electrical signal limits a rate of change of generation of the hydrogen ions, for example relative to a non-varying voltage, of equivalent magnitude to the electrical signal's peak amplitude, across the pair of electrodes provided by a direct current source.

Alternatively or additionally, the variation of the electrical signal may promote hydrogen ion neutralization, for example by the electrical signal comprising or being defined by a bidirectional waveform supplied to the electrodes. The neutralization promotion provided by such a bidirectional waveform will be explained in more detail herein below.

In some embodiments, the oral care device comprises an actuator. Such an actuator may, for example, move a cleaning element for cleaning inside the subject's oral cavity.

The movement of the actuator, for example causing movement of a brush head and/or controlling a pump of an irrigator included in the oral care device, may thus be provided alongside the hydrogen ion generation, with concomitant tartar removal enhancement.

In embodiments in which the oral care device includes the actuator, the actuator may be configured to move in accordance with the electrical signal.

The term "move in accordance with the electrical signal" may mean in this context that an actuator control signal provided by the control arrangement to control the movement of the actuator and the electrical signal are the same as each other, or one of the actuator control signal and the electrical signal is based on the other of the actuator control signal and the electrical signal.

Movement of the actuator in accordance with the electrical signal provided to the electrodes may, in certain embodiments, facilitate a particular cleaning movement, for example a reciprocating movement, being provided by the actuator, in addition to the electrical signal assisting to control hydrogen ion generation at the pair of electrodes.

In some embodiments, the actuator control signal is in phase with the electrical signal. Thus, the movement of the actuator may be synchronized with the electrical signal provided to the electrodes.

Alternatively, a phase difference may be deliberately defined between the actuator control signal and the electrical signal. Thus, the electrical signal can be desynchronized from the actuator control signal, and thus desynchronized from movement of the actuator, e.g. brush head motion/reciprocation.

In some embodiments, one or both of the electrodes is or are mechanically coupled to the actuator such that the movement of the actuator causes movement of the electrode(s).

In such embodiments, the actuator movement may result in an increased effective area, in other words footprint, in which the electrochemical treatment provided by the electrodes is implemented.

Alternatively or additionally, the oral care device may include at least one cleaning element for mechanically cleaning inside the subject's oral cavity.

In some embodiments, the at least one cleaning element is mechanically coupled to the actuator such that the movement of the actuator causes movement of the at least one cleaning element. The resulting combination of mechanical dental tartar break-up and pH-assisted dissolution of the dental tartar may be particularly effective, and may, for instance, also benefit from being implementable by the subject using the oral care device in their own home.

The at least one cleaning element may comprise bristles for brushing inside the subject's oral cavity. Alternatively or additionally, the at least one cleaning element may comprise a cup formed from a resilient material for rubbing against surfaces inside the subject's oral cavity. Such a cup may be, for instance, a so-called prophy cup.

Alternatively or additionally, the oral care device may include at least one cleaning element for fluidically cleaning inside the subject's oral cavity.

In such embodiments, the at least one cleaning element may be arranged such that said movement of the actuator causes fluid delivery from the at least one cleaning element.

In such embodiments, the actuator control signal may be, for example, an electrical switch signal of a pump of an oral irrigator.

In some embodiments, the electrical signal comprises a periodic variation in at least one of amplitude, phase and frequency. This may assist to control the build-up of hydrogen ions, and thus assist in controlling the local pH.

Alternatively or additionally, the electrical signal comprises a bidirectional waveform. Alternating the polarity of the electrodes via such a bidirectional waveform may assist to limit the progression of a low pH "front" emanating from the electrodes, and stabilize the pH. This is because, following a change in polarity, previously generated hydrogen ions at one of the electrodes may be neutralized by currently generated hydroxide ions at the same electrode.

In some embodiments, a positive peak amplitude of the bidirectional waveform is different from a negative peak amplitude of the bidirectional waveform. This may result in a continuous, but controlled increase or decrease in pH at a particular location.

In some embodiments, one of the positive and negative peak amplitudes is at least 1.1 times, such as about 1.5 times, the other of the positive and negative peak amplitudes.

In one set of embodiments, the electrical signal is a continuous waveform, for example a sine wave or square wave. Such a continuous waveform can be regarded as providing a continuously varying electrical signal.

In another set of embodiments, the electrical signal is a pulsed electrical signal. Such a pulsed electrical signal may result in a more gentle and controlled change of pH compared to, for example, the case of a non-varying voltage across the pair of electrodes.

In some embodiments, a duty cycle of the pulsed electrical signal is less than or equal to 90%, preferably less than or equal to 75%, most preferably less than or equal to 50%.

The duty cycle is the percentage of the ratio of pulse duration, or pulse width, to the total period of the waveform. The associated smaller total current "on" time associated with the pulsed electrical signal relative to a non-varying voltage across the pair of electrodes can assist to limit the progression of the above-mentioned low pH "front".

In some embodiments, the electrical (current or voltage) signal is both pulsed and bipolar. In such embodiments, a duty cycle associated with a positive component of the bipolar electrical signal may be the same as or different to a duty cycle associated with a negative component of the bipolar electrical signal.

In at least some embodiments, the control arrangement is configured to provide the electrical signal to the pair of electrodes for a predetermined time period before terminating the provision of the electrical signal to the pair of electrodes. Such a predetermined time period in combination with the variation of the electrical signal may assist to control build-up of hydrogen ions generated by the electrodes.

This predetermined time period may be the same as or different from a (further) predetermined time period during which the actuator moves in accordance with the electrical signal.

For example, the further predetermined period during which the actuator moves in accordance with the electrical signal may be longer than the predetermined period during which the electrical signal is provided to the pair of electrodes.

Mechanical cleaning via the actuator movement, for instance, can accordingly continue after the hydrogen ion generation has ceased due to termination of the predetermined time period.

In some embodiments, the pair of electrodes are insertable into the subject's oral cavity, and the aqueous solution comprises the subject's saliva and/or one or more oral care agents. This may provide a relatively physically simple way of providing a lower pH proximal to the subject's teeth.

Such oral care agent(s) may be at least one selected from a toothpaste, mouthrinse and a whitening agent.

It is noted, more generally, that the aqueous solution may, as well as the water, include one or more types of ions.

In some embodiments, the oral care device comprises a cleaning unit, such as a cup, for inserting into the subject's oral cavity. The cleaning unit may have a recess in which one or both of the pair of electrodes is or are mounted. In this manner, the cleaning unit may assist to protect the pair of electrodes and minimize the risk of the pair of electrodes unintentionally contacting tissue within the subject's oral cavity.

In some embodiments, the pair of electrodes are arranged with one of the electrodes defining an outer electrode which at least partially surrounds the other of the electrodes that defines an inner electrode.

As an alternative or in addition to one of the electrodes defining an outer electrode which at least partially surrounds an inner electrode, the pair of electrodes may be interdigitated.

The term "interdigitated" in this context may mean that each of the electrodes has a plurality of electrode members spaced apart from each other, with electrode members of one of the pair of electrodes being arranged in spaces between electrode members of the other of the pair of electrodes.

This may provide a particularly spatially efficient arrangement of the pair of electrodes, particularly when the pair of electrodes is to be included in a brush head and/or mounted within a cleaning unit, e.g. a cup, where space may be limited. The interdigitated electrodes may thus assist to simplify the architecture of the oral care device and limits the required electrical contacts to drive electrodes.

Moreover, in combination with the above-described electrical signal, such an interdigitated arrangement of the electrodes may assist in terms of defining the electrolytic footprint provided by the device.

In some embodiments, the cleaning unit, for example cup, is formed from a resilient material for rubbing against surfaces inside the subject's oral cavity. Such a cup may be, for instance, a so-called prophy cup.

In embodiments in which the oral care device comprises at least one cleaning element mechanically coupled to an actuator such that the movement of the actuator causes movement of the at least one cleaning element, the at least one cleaning element can include the cup.

For example, the at least one cleaning element can include the cup in addition to bristles.

More generally, the oral care device may comprise, or be, a toothbrush, a mouthpiece, an irrigator, or a professional dental instrument operable by a dental practitioner or oral hygienist. Particular mention is made of an oral care device in the form of a toothbrush.

According to another aspect there is provided a method of operating an oral care device comprising a pair of electrodes for contacting an aqueous solution, the method comprising providing an electrical signal to the pair of electrodes to cause generation of hydrogen ions from water in the aqueous solution to deliver within a subject's oral cavity, a variation of the electrical signal controlling said generation of hydrogen ions.

The device employed in the method may be according to any of the embodiments described herein. In particular, the electrical signal may be provided by the control arrangement included in the device.

More generally, embodiments described herein in relation to the method may be applicable to the device, and embodiments described herein in relation to the device may be applicable to the method.

### BRIEF DESCRIPTION OF THE DRAWINGS

Examples of the invention will now be described in detail with reference to the accompanying drawings, in which:
FIG. 1 provides a schematic depiction of dental tartar layered on a pellicle layer of a tooth;
FIG. 2 provides graphs showing solubility of crystalline phases of calcium phosphate as a function of pH;
FIG. 3 schematically depicts a device according to an example;
FIG. 4 schematically depicts a device according to another example;
FIG. 5 schematically depicts a device according to a still another example;
FIG. 6A shows a circuit for providing a unipolar pulsed electrical signal;
FIG. 6B shows a bipolar signal (upper pane), and a unipolar pulsed electrical signal (lower pane) provided by the circuit shown in FIG. 6A;
FIG. 7 schematically depicts actuator-driven movement of electrolysis electrodes according to an example;
FIG. 8 schematically depicts an oral care device according to an example;
FIG. 9 shows part of an oral care device according to another example;
FIG. 10 provides graphs of tartar and enamel dissolution depth in 30 seconds vs voltage across electrolysis electrodes;
FIG. 11 schematically depicts a 2D simulation setup including two electrolysis electrodes;
FIG. 12A provides a graph of a non-varying voltage;
FIG. 12B provides graphs of pH vs time at different points proximal to the electrodes shown in FIG. 11 when the electrical signal shown in FIG. 12A is provided to the electrodes;
FIG. 13A provides a graph of an electrical signal according to a first example;
FIG. 13B provides graphs of pH vs time at different points proximal to the electrodes shown in FIG. 11 when the electrical signal shown in FIG. 13A is provided to the electrodes;
FIG. 14A provides a graph of an electrical signal according to a second example;
FIG. 14B provides graphs of pH vs time at different points proximal to the electrodes shown in FIG. 11 when the electrical signal shown in FIG. 14A is provided to the electrodes;
FIG. 15 provides a graph of an electrical signal according to a third example; and
FIG. 16 provides a graph of an electrical signal according to a fourth example.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Provided is an oral care device for dental tartar removal. The oral care device comprises a control arrangement configured to provide an electrical signal, for example a time-varying electrical signal. The oral care device also comprises a pair of electrodes for contacting an aqueous solution. The pair of electrodes are each connected to the control arrangement and configured to, responsive to said electrical signal, generate hydrogen ions from water in the aqueous solution to deliver within a subject's oral cavity, in particular to the subject's teeth. A variation of the electrical signal controls said generation of hydrogen ions. The thus generated hydrogen ions can lower a pH local to the subject's teeth and thereby assist to dissolve tartar thereon. The variation, for example time-variation, of the electrical signal may provide control over the generation of hydrogen ions which contrasts with, for instance, an uncontrolled generation of hydrogen ions via a non-varying voltage across the pair of electrodes provided by a direct current source. Further provided is a method of operating such an oral care device.

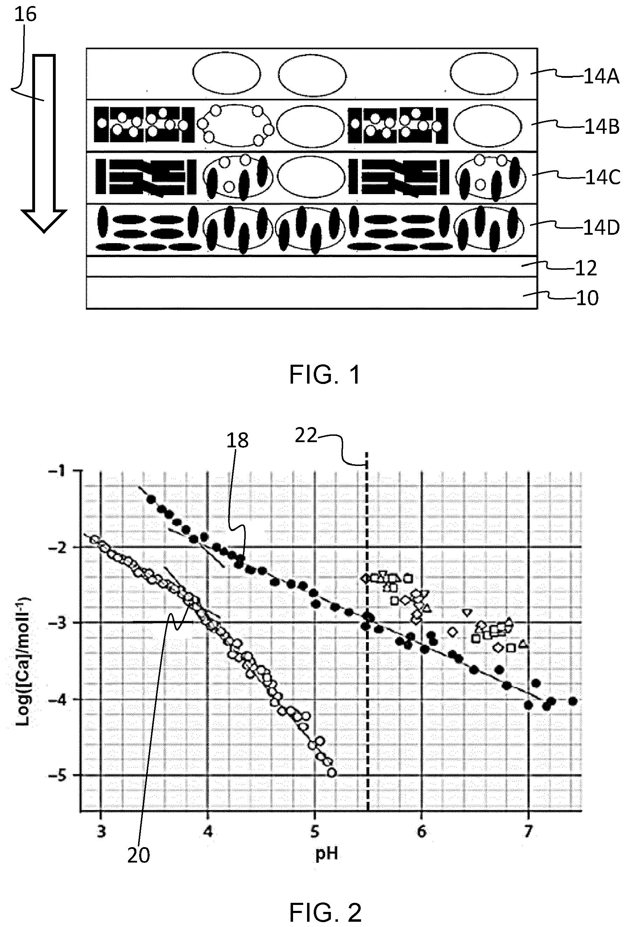

FIG. 1 schematically depicts an enamel layer 10 of a tooth, and a pellicle layer 12 on the enamel layer 10. FIG. 1 also shows dental tartar 14A, 14B, 14C, 14D layered on the pellicle layer 12.

The dental tartar 14A-D shown in FIG. 1 includes several different calcium phosphate crystalline phases which have different aqueous solubilities. The dental tartar layer 14A furthest from the pellicle layer 12 comprises dicalcium phosphate dihydrate (DCPD), the layer 14B adjacent the dental tartar layer 14A comprises octacalcium phosphate (OCP), the layer 14D closest to the pellicle layer 12 and the layer 14C between the layer 14B and the layer 14D may comprise a relatively small quantity of hydroxyapatite (HAp). HAp is a key component of the enamel layer 10.

HAp has lower aqueous solubility than OCP, and OCP has lower aqueous solubility than DCPD, such that the relative and/or absolute aqueous solubility decreases in the direction of arrow 16. The absolute solubility of OCP and DCPD in water, is however low, and their proximity to the enamel layer 10, can therefore hamper removal of dental tartar, particularly by a subject in their own home.

The aqueous solubilities of the above-mentioned crystalline phases have been found to be pH dependent. In particular, dissolution in aqueous solution can be assisted by lowering the pH. FIG. 2 provides graphs 18, 20 showing solubility of tartar-comprising crystalline phases of calcium phosphate as a function of pH. FIG. 2 generally shows that the concentration of calcium ions dissolved in solution increases as the pH decreases for both OCP (graph 18), and HAp (graph 20); at pH 5.5 the solubility of OCP being almost 2 orders of magnitude higher than HAp.

A pH at which dental enamel 10 may start to dissolve is also denoted by the dotted line 22 in FIG. 2. This pH may be around pH 5.5, clinically considered the critical in-mouth pH at which dental enamel dissolves (accepted as a threshold).

The present disclosure is partly based on the realization that by controlling the pH local to the teeth to be less than 7 but equal to or greater than 5.5, the OCP and DCPD of dental tartar 14, in particular early stage dental tartar, can be dissolved but with minimal or no damage to dental enamel 10. This is because such a pH range may minimize dissolution of HAp in the dental enamel 10, as shown in FIG. 2.

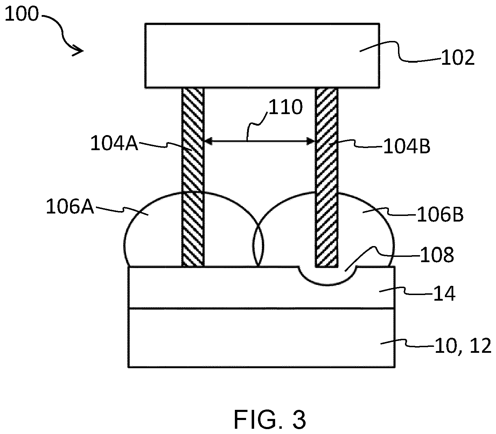

FIG. 3 schematically depicts a device 100 according to an example. The device 100 comprises a control arrangement 102 configured to provide an electrical signal. The device 100 also comprises a pair of electrodes 104A, 104B for contacting an aqueous solution. The pair of electrodes 104A, 104B are each connected to the control arrangement 102 and configured to, responsive to the electrical signal, generate hydrogen ions from water in the aqueous solution.

Implicit in this generation of hydrogen ions, in other words H⁺ ions, from water in the aqueous solution is that a potential difference between the electrodes 104A, 104B resulting from the electrical signal is sufficient for water electrolysis to take place.

The electrodes 104A, 104B can be made of any suitable electrically conductive material capable of electrochemical generation of hydrogen ions via water electrolysis. For example, the electrodes 104A, 104B may be selected from platinum electrodes, platinum-clad electrodes, such as platinum clad titanium electrodes, gold electrodes, gold-clad electrodes, carbon electrodes, such a graphite electrodes, and polymer-based electrodes in which the polymer is embedded with the conductive material, for example with a conductive material selected from platinum, gold, or carbon, e.g. graphite.

In some embodiments, each of the pair of electrodes 104A, 104B is a non-sacrificial electrode.

The term "non-sacrificial electrode" may mean that the electrodes 104A, 104B are each formed of a conductive material which does not, or only negligibly, dissociates into ions in response to the electrical signal.

In at least some embodiments, the pair of electrodes 104A, 104B are insertable into the subject's oral cavity, with the aqueous solution comprising the subject's saliva and/or one or more oral care agents, such as toothpaste, mouthrinse and/or a whitening agent. This may provide a relatively physically simple way of providing a lower pH proximal to the subject's teeth.

Referring again to FIG. 3, the following reaction may occur at the electrode 104A when this electrode is operated as a cathode: 2H₂O + 2e⁻ ↔ 2OH⁻ + H₂. (or, 2H⁺+ 2e⁻ ↔ H₂)

This production of hydroxide ions (or consumption of hydrogen ions) may raise the pH proximal to the electrode 104A. Such a local increase in pH, denoted in FIG. 3 by area 106A around electrode 104A, may have less influence on dental tartar 14 solubility than the local decrease in pH proximal to the electrode 104B, denoted by area 106B around electrode 106B, when the latter is operated as an anode: H₂O ↔ 2H⁺ + 0.5O₂ + 2e⁻ (or, 2OH⁻ ↔ O₂ + 2H⁺ + 2e⁻). This is because dental tartar dissolution, as represented in FIG. 3 by a tartar dissolution spot 108 proximal to the electrode 104B, may be assisted by lowering the pH, in other words increasing the hydrogen ion concentration, as previously explained.

The pair of electrodes 104A, 104B may be spaced apart from each other by any suitable distance 110, such as a distance 110 greater than 50 µm, for example between 50 µm and 1 mm.

A distance 110 greater than 50 µm may assist to suppress immediate neutralization of the hydrogen ions generated at the anode by the hydroxide ions generated at the cathode.

A half-distance (L/2) of, for instance, 25 µm may be covered by the pH front in ~0.07 s (L²/4D, where D = 9^{∗} 10⁻⁹ m²/s, the diffusion coefficient of H⁺ ions). This lower limit of 50 µm may mean that a total treatment time is >0.07 s. This total treatment time is the time period, e.g. predetermined time period, during which the electrical signal is provided to the pair of electrodes 104A, 104B (and so is not the current on/off time in a pulse in embodiments in which the electrical signal is pulsed).

More generally, it has been found that employing a non-varying voltage across the pair of electrodes provided by a direct current source can provide relatively uncontrolled generation of hydrogen ions. An electrical signal, for example time-varying electrical signal can, by contrast, assist to enhance the control that can be exerted over the generation of hydrogen ions, and thus the local pH to which the subject's teeth are exposed. This is explained in more detail herein below.

The device 100 may comprise, or be, a toothbrush, a mouthpiece, an irrigator, or a professional dental instrument operable by a dental practitioner or oral hygienist. Particular mention is made of an oral care device 100 in the form of a toothbrush.

It is noted that a toothbrush, a mouthpiece, and an irrigator can be regarded as examples of a personal care oral device 100. Such a personal care oral device 100 may be used by the subject him/herself in their own home.

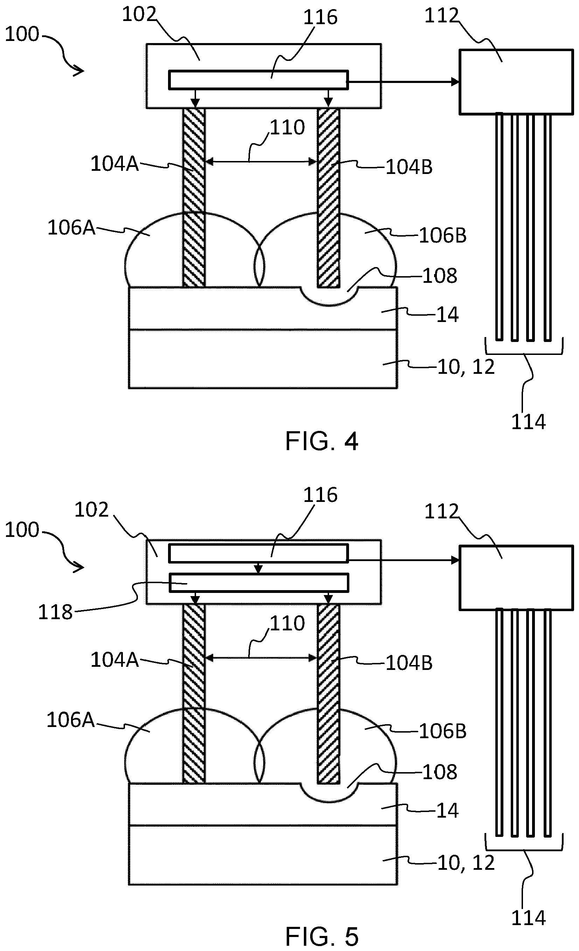

In some embodiments, such as that shown in FIG. 4, the device 100 comprises an actuator 112 connected to the control arrangement 102. In such embodiments, the actuator 112 may be configured to move in accordance with the electrical signal generated by the control arrangement 102.

The term "move in accordance with the electrical signal" may mean in this context that an actuator control signal provided by the control arrangement 102 to control the movement of the actuator 112 and the electrical signal are the same as each other, or one of the actuator control signal and the electrical signal is based on the other of the actuator control signal and the electrical signal.

The actuator control signal may, for example, be in phase with the electrical signal, or a phase difference may be defined between the actuator control signal and the electrical signal.

The movement of the actuator 112, for example causing movement of a brush head of the device 100 in the form of an oral care device 100, may thus be provided alongside the hydrogen ion generation, with concomitant tartar removal enhancement.

Movement of the actuator 112 in accordance with the electrical signal provided to the electrodes 104A, 104B may, in certain embodiments, facilitate a particular cleaning movement, for example a reciprocating movement, being provided by the actuator 112, in addition to the electrical signal assisting to control hydrogen ion generation at the pair of electrodes 104A, 104B.

Any suitable type of actuator 112 can be contemplated, for example a motor, such as a reciprocating motor (not visible in the Figures).

In some embodiments, such as that shown in FIG. 4, the device 100 comprises at least one cleaning element 114 for mechanically cleaning inside the oral cavity, which at least one cleaning element 114 is mechanically coupled to the actuator 112 such that the movement of the actuator 112 causes movement of the at least one cleaning element 114.

Any suitable type of cleaning element can be contemplated. In the non-limiting example shown in FIG. 4, the at least one cleaning element 114 comprises bristles for brushing inside the subject's oral cavity. In such an embodiment, the dental tartar removal facilitated by the hydrogen ions generation may be enhanced by the bristles movement resulting from the movement of the actuator 114 in accordance with the electrical signal.

A reciprocating movement of the actuator 112, e.g. the above-mentioned reciprocating motor, may be provided in accordance with a periodically varying electrical signal.

In such embodiments, the reciprocating movement of the actuator 112 may cause the cleaning element(s) 114, for example bristles, to correspondingly move in a reciprocating manner.

Such a periodic electrical signal may also assist to control the local pH, in particular so as to help reduce the risk of the local pH at one or both of the electrodes 104A, 104B from decreasing below a certain pH level, as will be explained in more detail herein below.

This combination of mechanical dental tartar break-up and low local pH-assisted dissolution of the dental tartar may be particularly effective, and may, for instance, also benefit from being implementable by the subject using the device 100 in their own home.

In some non-limiting examples, the at least one cleaning element 114 comprises, as an alternative or in addition to bristles, a cup formed from a resilient material for rubbing against surfaces inside the subject's oral cavity. Such a cup may be, for instance, a so-called prophy cup.

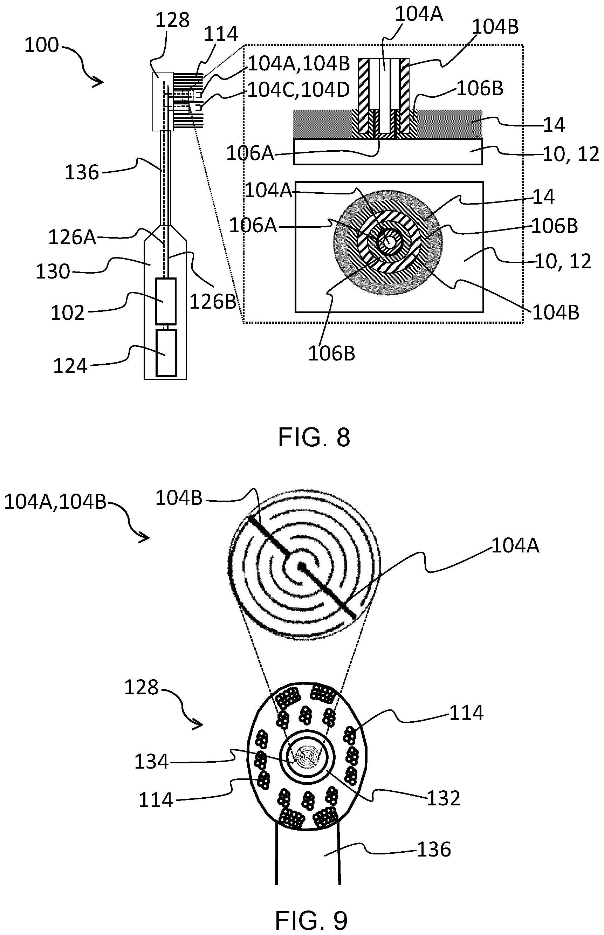

In embodiments in which a cup formed from a resilient material is included in the oral treatment device 100, irrespective of whether such a cup is arranged to be moved by an actuator 114, one or both of the pair of electrodes 104A, 104B may be mounted inside the cup. An example of this will be explained below with reference to FIG. 9.

In certain embodiments, such as that shown in FIG. 4, the control arrangement 102 includes drive train circuitry 116 configured to provide the actuator control signal to control the actuator 112 and provide the electrical signal to the pair of electrodes 104A, 104B.

In such embodiments, the actuator control signal may be the same as the electrical signal, so as to provide an advantageously simple and cost-effective electrical design.

In alternative embodiments, such as that shown in FIG. 5, the control arrangement 102 comprises, in addition to the drive train circuitry 116 configured to provide the actuator control signal, electrode control circuitry 118 configured to convert the drive train control signal to the electrical signal for providing to the pair of electrodes 104A, 104B.

The electrode control circuitry 118 can, for example, include particularly only passive electronic components, such as diode(s), resistor(s) and/or capacitor(s), arranged to convert the drive train control signal to the electrical signal.

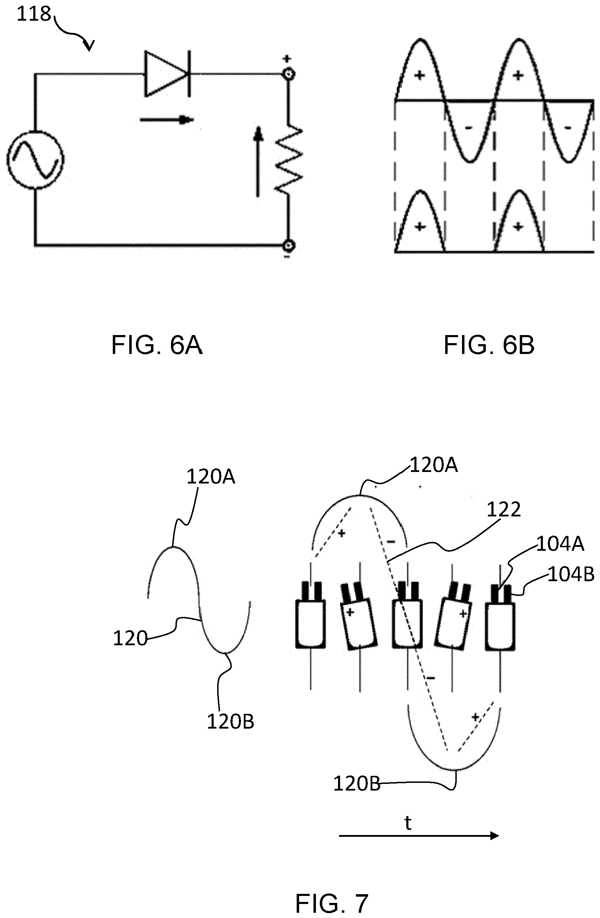

An example of such electrode control circuitry 118 is shown in FIG. 6A. In this example, the diode converts the alternating current signal shown in the upper pane of FIG. 6B to a rectified output waveform, in which the negative half-cycle is absent, as shown in the lower pane of FIG. 6B.

The drive train circuitry 116 can be implemented in any suitable manner, such as by the drive train circuitry 116 comprising a microcontroller, e.g. motor microcontroller, configured to generate the actuator control signal.

It is also noted that the alternating current signal of the example shown in FIGs. 6A and 6B need not be provided via drive train circuitry 116, and in other examples may be provided by a power supply, for example, a mains power source (not visible in the Figures) arranged to power the device 100.

In such examples, the control arrangement 102 may include the electrode control circuitry 118 to provide a unipolar pulsed signal (as shown in FIG. 6B, lower pane) from an alternating current signal provided by the power supply, with the drive train control circuitry 116 being omitted.

In some embodiments, such as that shown in FIG. 7, one or both of the electrodes 104A, 104B is or are mechanically coupled to the actuator 112 such that the movement of the actuator 112 causes movement of the electrode(s) 104A, 104B.

In the non-limiting example shown in FIG. 7, the electrical signal 120 is a periodic bipolar electrical signal varying with time, t.

In a positive polarity half-cycle 120A of the electrical signal 120 shown in FIG. 7, the actuator 112, and thus the electrodes 104A, 104B, exhibit a first movement. In a negative polarity half-cycle 120B of the electrical signal 120, the actuator 112, and thus the electrodes 104A, 104B, exhibit a second movement mirroring the first movement. The gradient/slope of the electrical signal 120 is represented in FIG. 7 by the dotted lines 122.

This may be implemented by the control arrangement 102 providing an actuator control signal to control the actuator 112 that is in phase with the electrical signal provided to the electrodes 104A, 104B.

The actuator movement may result in an increased effective area, in other words footprint, in which the electrochemical treatment provided by the electrodes 104A, 104B is implemented. Moreover, the alternating polarity may mean that the hydrogen ion generation is implemented at both of the electrodes 104A, 104B but sequentially, thereby further assisting to increase the effective area in which the electrochemical treatment is provided. In addition, the periodic electrical signal may also assist to control the local pH, in particular so as to reduce the risk of the local pH at the electrodes 104A, 104B decreasing below a certain pH level, as will be explained in more detail herein below.

In alternative embodiments, the control arrangement 102 may be configured to provide an actuator control signal to control the actuator 112, with a phase difference being defined between the actuator control signal and the electrical signal provided to the electrodes 104A, 104B.

Thus, the electrical signal can be desynchronized from the actuator control signal, and thus desynchronized from movement of the actuator 112, e.g. brush head motion, such as brush head reciprocation.

Such a phase difference can be introduced in any suitable manner, such as via one or more capacitor(s), inductor(s), etc. Thus, a relatively small number of electrical components (in addition to those for providing the actuator control signal) may be required to provide the phase difference.

In some embodiments, the control arrangement 102 comprises, in addition to drive train circuitry 116 configured to provide the actuator control signal, electrode control circuitry 118 configured to convert the actuator control signal to the electrical signal provided to the electrodes 104A, 104B such that the phase difference is deliberately defined between the actuator control signal and the electrical signal.

In some embodiments, such as that shown in FIG. 8, the device 100 in the form of an oral care device 100 comprises a plurality of pairs of electrodes 104A, 104B; 104C, 104D. In such embodiments, the control arrangement 102 may be configured to provide the electrical signal to each of the plurality of pairs of electrodes 104A, 104B; 104C, 104D.

The oral care device 100 may be powered in any suitable manner, such as via a mains source of electricity and/or by one or more batteries 124. In embodiments in which the oral care device 100 is powered by one or more batteries 124, the control arrangement 102 may be configured to generate the electrical signal, for example a current or voltage signal, from a direct current resulting from the one or more batteries 124.

A first electrical connection 126A may connect the control arrangement 102 to one of the electrodes 104A of the pair of electrodes 104A, 104B, with a second electrical connection 126B connecting the control arrangement 102 to the other electrode 104B of the pair of electrodes 104A, 104B.

In the non-limiting example shown in FIG. 8 in which two pairs of electrodes 104A, 104B; 104C, 104D are included in the oral care device 100, the first electrical connection 126A connects the control arrangement 102 to the electrodes 104A and 104C, and the second electrical connection 126B connects the control arrangement 102 to the electrodes 104B and 104D.

In some embodiments, such as that shown in FIG. 9 the oral care device 100 comprises a toothbrush including a brush head 128 attached to a handle 130. In such embodiments, the pair of electrodes 104A, 104B are preferably included in the brush head 128, with the control arrangement 102 being included in the handle 130.

In the non-limiting example shown in FIG. 8, the one or more batteries 124 are housed, together with the control arrangement 102, within the handle 130.

In some embodiments, the brush head 128, including the pair of electrodes 104A, 104B, is detachably coupled to the handle 130 to enable replacement of the brush head 128 without having to replace the control arrangement 102 included in the handle 130. In such embodiments, the first and second electrical connections 126A, 126B between the control arrangement 102 included in the handle 130 and the pair of electrodes 104A, 104B included in the (replacement) brush head 128 may be established at the same time as the brush head 128 is attached, for example snap-fitted or push-fitted, to the handle 130.

In the non-limiting example shown in FIG. 8, each of the plurality of pairs of electrodes 104A, 104B; 104C, 104D are included in the brush head 128.

In embodiments in which the oral care device 100 is or comprises a toothbrush, the brush head 128 may comprise at least one cleaning element 114, such as the above-described bristles and/or the cup formed from a resilient material for rubbing against surfaces inside the subject's oral cavity.

In some embodiments, such as that shown in FIG. 8, the at least one cleaning element 114 comprises bristles, and the bristles extend further from the brush head 128 than each of the pair of electrodes 104A, 104B, such that pair of electrodes 104A, 104B are both recessed relative to the bristles.

The at least one cleaning element 114, and in some cases the brush head 128 as a whole, may be moved by an actuator 112, as previously described.

In embodiments, in which the at least one cleaning element 114 comprises the cup formed from a resilient material, one or both of the pair of electrodes 104A, 104B may be mounted inside the cup.

In embodiments, such as that shown in FIG. 8, in which a plurality of pairs of electrodes 104A, 104B; 104C, 104D is included in the oral care device 100, the oral care device 100 may include a plurality of cups. In such embodiments, one or both electrodes 104A, 104B; 104C, 104D of each pair may be mounted inside a respective cup of the plurality of cups.

The electrodes 104A, 104B can be arranged relative to each other in any suitable manner. In some embodiments, the electrodes 104A, 104B are arranged with one of the electrodes 104B defining an outer electrode 104B which at least partially surrounds the other of the electrodes 104A that defines an inner electrode 104A. An example of this is shown in FIG. 8.

FIG. 8 also shows the area 106A around the electrode 104A, and the area 106B around the electrode 104B. In one of these areas 106A, 106B, depending on the electrical signal, the local pH may be increased due to hydroxide ion generation while in the other of these areas 106B, 106A the local pH may be decreased due to hydrogen ion generation, as previously described in relation to FIG. 3.

As an alternative or in addition to one of the electrodes 104B defining an outer electrode 104B which at least partially surrounds an inner electrode 104A, the pair of electrodes 104A, 104B may be interdigitated.

The term "interdigitated" in this context may mean that each of the electrodes 104A, 104B has a plurality of electrode members spaced apart from each other, with electrode members of one of the pair of electrodes 104A, 104B being arranged in spaces between electrode members of the other of the pair of electrodes 104B, 104A.

This may provide a particularly spatially efficient arrangement of the pair of electrodes 104A, 104B, particularly when the pair of electrodes 104A, 104B is to be included in a brush head 128 and/or mounted within a cup where space may be limited.

A non-limiting example of such an interdigitated arrangement of the pair of electrodes 104A, 104B is depicted in FIG. 9.

Similarly to the non-limiting example shown in FIG. 8, cleaning elements 114 comprising bristles are included in the oral care device 100 shown in FIG. 9. In the latter non-limiting example, a plurality of peripheral tufts of bristles are arranged around a centrally positioned pair of interdigitated electrodes 104A, 104B.

In some embodiments, such as that shown in FIG. 8, the oral care device 100 comprises a cup 132 for inserting into the subject's oral cavity, with the cup 132 having a recess 134 in which one or both of the pair of electrodes 104A, 104B is or are mounted. In this manner, the cup 132 may assist to protect the pair of electrodes 104A, 104B and minimize the risk of the pair of electrodes 104A, 104B unintentionally contacting tissue within the subject's oral cavity.

The cup 132 may be formed of any suitable, for example electrically insulating, material. In some embodiments, the cup 132 is formed from a resilient material for rubbing against surfaces inside the subject's oral cavity, as previously described.

The cup 132 formed from the resilient material may flare and flex to contours of the subject's teeth to aid in selective stain removal and cleaning between teeth.

Any suitable resilient material can be considered for the cup 132, such as silicone rubber.

In some embodiments, such as that shown in FIGs. 8 and 9, the brush head 128 is attached, for example detachably coupled, to a handle 130 of the oral care device 100, in this case in the form of a toothbrush, via a neck 136. Such a neck 136 may extend between an upper end of the handle 130 and a lower end of the brush head 128.

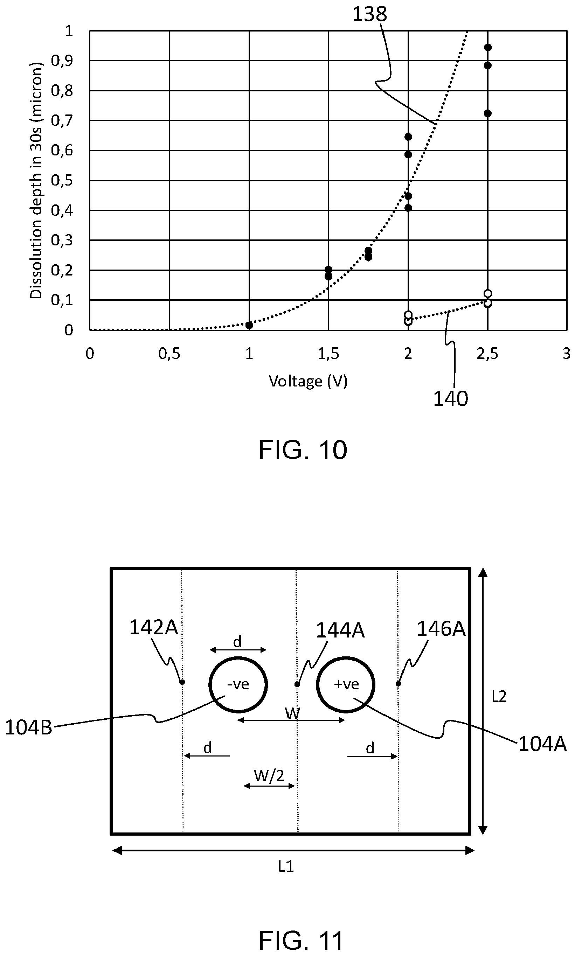

FIG. 10 provides a graph 138 of tartar dissolution depth in 30 seconds vs continuous DC voltage across the pair of electrodes 104A, 104B. As the voltage increases, the associated local decrease in pH caused by the hydrogen ion generation results in dissolution of dental tartar to a greater depth. However, FIG. 10 also provides a graph 140 of dental enamel dissolution depth in 30 seconds vs continuous DC voltage across the pair of electrodes 104A, 104B which shows that relatively low pH conditions caused by relatively high voltages can cause dissolution of dental enamel.

FIG. 11 schematically depicts a 2D simulation setup including two electrolysis electrodes 104A, 104B in a 0.1 M KCl solution (to correspond to experimental measurements, shown in Fig. 10. Saliva-toothpaste mixture also has conductivity of similar order of magnitude), with d = 145 µm, and w = 500 µm. L1 and L2 represent domain boundaries. This 2D simulation setup was used to assess various electrical signals in exemplary embodiments.

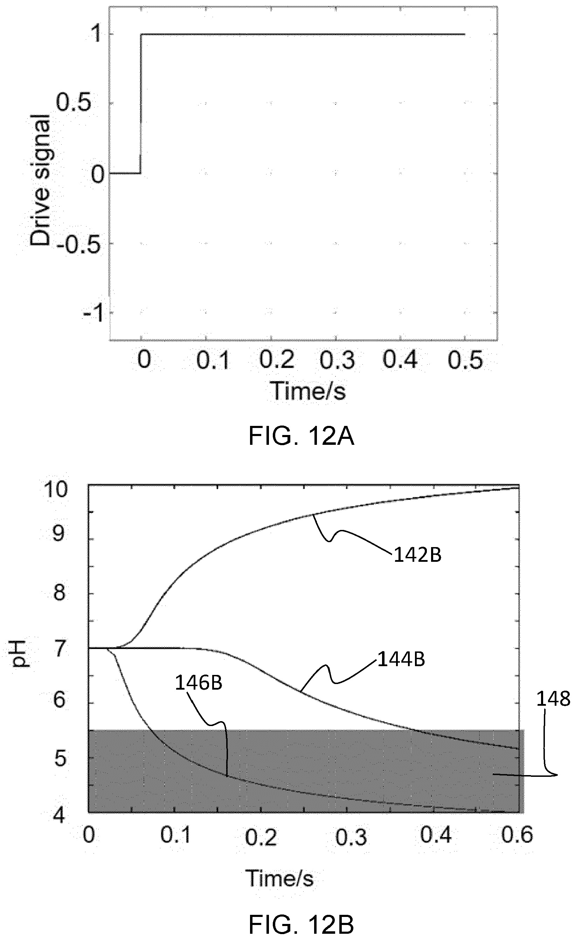

FIG. 12A provides a graph of a non-varying voltage (step signal) across the pair of electrodes provided by a direct current source, and FIG. 12B provides graphs 142B, 144B, 146B of pH vs time at respective points 142A, 144A, 146A proximal to the electrodes 104A, 104B shown in FIG. 11 when the electrical signal shown in FIG. 12A is provided to the electrodes 104A, 104B.

Whilst the application of a direct current signal leads to a relatively large change and strong drift in pH, a relatively rapid/uncontrolled decrease towards a pH less than pH 5.5 (see region 148 in FIG. 12B) can be observed in graph 146B (corresponding to point 146A in FIG. 11).

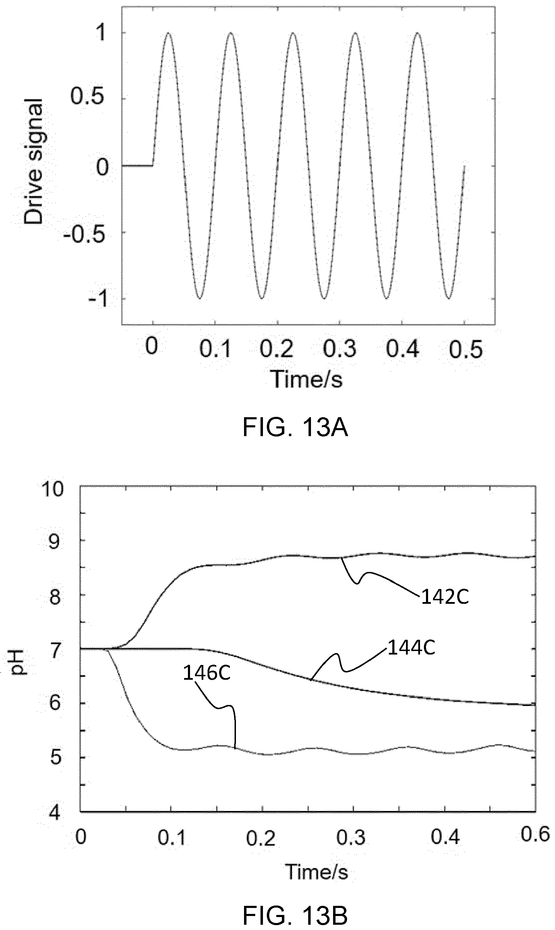

FIG. 13A provides a graph of an electrical signal according to a first example in which the electrical signal is periodic and bipolar, and FIG. 13B provides graphs 142C, 144C, 146C of pH vs time at respective points 142A, 144A, 146A proximal to the electrodes 104A, 104B shown in FIG. 11 when the electrical signal shown in FIG. 13A is provided to the electrodes 104A, 104B.

To enable comparison with FIGs. 13A and 13B, it is noted that the intensity of the non-varying voltage shown in FIG. 12A is normalized.

The electrical signal shown in FIGs. 13A and 13B can be seen to induce relatively rapid stabilization of pH and less overall change of pH, having stabilized around pH 5. Thus, the varying of the electrical signal can enhance the control that can be exerted over the generation of hydrogen ions, and thus the local pH to which the subject's teeth are exposed.

In some embodiments, such as that shown in FIGs. 13A and 13B, the electrical signal comprises a periodic variation in amplitude. This may assist to control the build-up of hydrogen ions, and thus assist in controlling the local pH.

Alternatively or additionally, the electrical signal comprises, or is, a bidirectional waveform. An example of this is shown in FIG. 13A. Alternating the polarity of the electrodes 104A, 104B via such a bidirectional waveform may assist to limit the progression of a low pH "front" emanating from the electrodes 104A, 104B, and stabilize the pH. This is because, following a change in polarity, previously generated hydrogen ions at one of the electrodes 104A, 104B may be neutralized by currently generated hydroxide ions at the same electrode 104A, 104B.

In some embodiments, such as that shown in FIGs. 13A and 13B, the electrical signal comprises, or is, a continuous waveform. In particular, FIG. 13A shows a continuous waveform in the form of a sine wave.

In alternative embodiments, the electrical signal comprises, or is, a pulsed electrical signal. Such a pulsed electrical signal can, for instance, be generated in the manner described above in relation to FIGs. 6A and 6B.

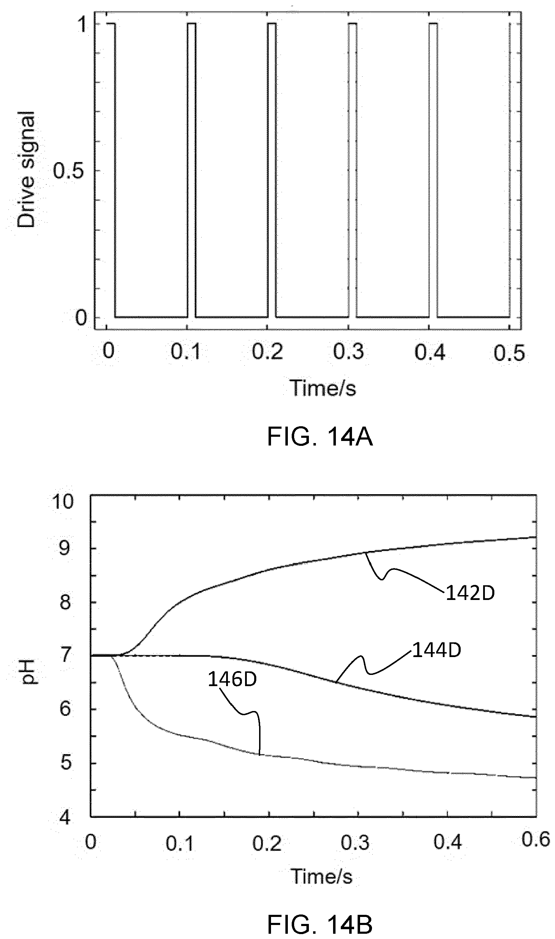

FIG. 14A provides a graph of an electrical signal according to a second example in which the electrical signal is periodic, pulsed (10 Hz, 10% duty cycle) and unipolar, and FIG. 14B provides graphs 142D, 144D, 146D of pH vs time at respective points 142A, 144A, 146A proximal to the electrodes 104A, 104B shown in FIG. 11 when the electrical signal shown in FIG. 14A is provided to the electrodes 104A, 104B.

In contrast to the non-varying voltage of FIGs. 12A and 12B in which the pH reaches an unsafe value of pH 4 relatively rapidly, the pulsed signal of FIGs. 14A and 14B results in a much more gentle and controlled change of pH reaching pH 4.8 after about 0.5 seconds.

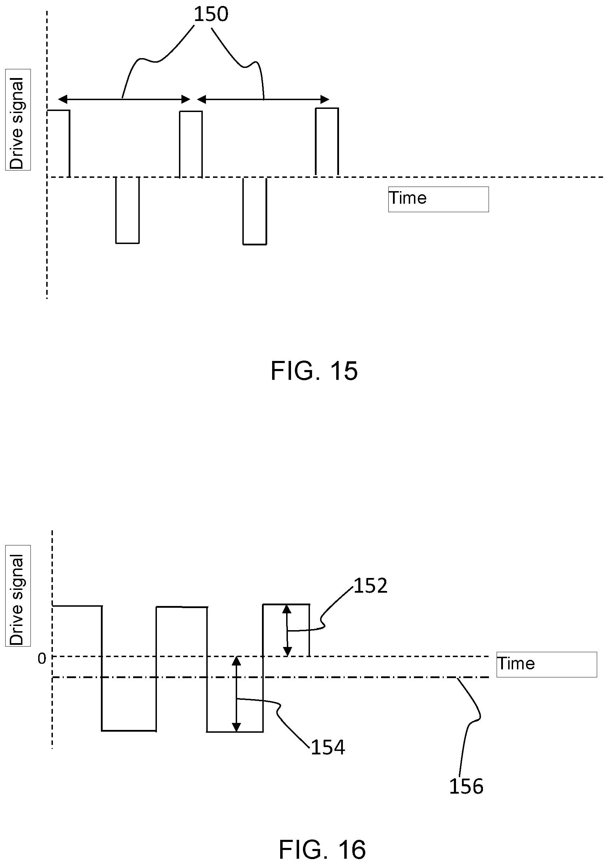

In some embodiments, and referring to FIG. 15, a duty cycle of the pulsed electrical signal is less than or equal to 90%, preferably less than or equal to 75%, most preferably less than or equal to 50%. The duty cycle is the percentage of the ratio of pulse duration, or pulse width, to the total period 150 of the waveform. The associated smaller total current "on" time associated with the pulsed electrical signal relative to the non-varying voltage of FIGs. 12A and 12B can assist to limit the progression of the low pH "front".

In some embodiments, such as that shown in FIG. 15, the electrical (current or voltage) signal is both pulsed and bipolar. The signal in each half cycle can be applied with a given duty cycle. The duty cycle is 50% in the non-limiting example shown in FIG. 15.

A duty cycle associated with a positive component of the bipolar electrical signal may be the same as or different to a duty cycle associated with a negative component of the bipolar electrical signal.

It is noted that in some embodiments, no duty cycle may be employed.

The electrical signal may, in some embodiments, be defined by asymmetric pulsing. Such an asymmetric electrical signal may assist to induce preferential pH change at a particular location.

In some embodiments, such as that shown in FIG. 16, the electrical signal is a bidirectional waveform, and a positive peak amplitude 152 of the bidirectional waveform is different from a negative peak amplitude 154 of the bidirectional waveform. This may result in a continuous, but controlled increase or decrease in pH at a particular location. Thus, the greater control provided by pulsing of the electrical signal relative to the application of a non-varying voltage may be combined with the limiting of creation of "extreme" pH zones near the electrode(s) 104A, 104B due to periodic neutralization provided by the changing polarity.

In some embodiments, one of the positive and negative peak amplitudes 152, 154 is at least 1.1 times the other of the positive and negative peak amplitudes 154, 152.

In the non-limiting example shown in FIG. 16, the negative peak amplitude 154 is 1.5 times the positive peak amplitude 152. The mean signal is represented in FIG. 16 by the dotted line 156.

In at least some embodiments, the control arrangement 102 is configured to provide the electrical signal to the pair of electrodes 104A, 104B for a predetermined time period before terminating the provision of the electrical signal to the pair of electrodes 104A, 104B. Such a predetermined time period in combination with the variation of the electrical signal may assist to control build-up of hydrogen ions generated by the electrodes 104A, 104B.

This predetermined time period may be the same as or different from a (further) predetermined time period during which the actuator 112 moves in accordance with the electrical signal. For example, the further predetermined period during which the actuator 112 moves in accordance with the electrical signal may be longer than the predetermined period during which the electrical signal is provided to the pair of electrodes 104A, 104B.

A method of operating a device comprising a pair of electrodes 104A, 104B for contacting an aqueous solution is also provided. The device may be according to any of the embodiments described herein. The method comprises providing an electrical signal to the pair of electrodes 104A, 104B to cause generation of hydrogen ions from water in the aqueous solution. A variation of the electrical signal controls said generation of hydrogen ions.

The electrical signal may be provided by the control arrangement 102 included in the device 100 described herein.

The above-described control arrangement 102 (and/or the drive train circuitry 116) can be implemented in numerous ways, with software and/or hardware, to perform the various required functions. A processor is one example of a control arrangement 102/drive train circuitry 116 which employs one or more microprocessors that can be programmed using software (e.g., microcode) to perform the functions. The control arrangement 102/drive train circuitry 116 may, however, be implemented with or without employing a processor, and also may be implemented as a combination of dedicated hardware to perform some functions and a processor, e.g. one or more programmed microprocessors and associated circuitry, to perform other functions.

Examples of controller components that may be employed in various embodiments of the present disclosure include, but are not limited to, conventional microprocessors, application specific integrated circuits (ASICs), and field-programmable gate arrays (FPGAs).

In some examples, the control arrangement 102/drive train circuitry 116 is associated with one or more storage media such as volatile and non-volatile computer memory such as RAM, PROM, EPROM, and EEPROM. The storage media can be encoded with one or more programs that, when executed on one or more processors and/or controllers, perform the required functions. Various storage media may be fixed within the control arrangement 102/drive train circuitry 116 or may be transportable, such that the one or more programs stored thereon can be loaded into the control arrangement 102/drive train circuitry 116.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. An oral care device (100) comprising:
a control arrangement (102) configured to provide an electrical signal; and
a pair of electrodes (104A, 104B) for contacting an aqueous solution, the pair of electrodes being connected to the control arrangement, the electrical signal and the pair of electrodes being configured such that at least one of the electrodes, responsive to said electrical signal, generates hydrogen ions from water in the aqueous solution to deliver within a subject's oral cavity, a variation of the electrical signal controlling said generation of hydrogen ions.

2. The oral care device (100) according to claim 1, wherein the variation of the electrical signal limits a rate of change of generation of the hydrogen ions and/or promotes hydrogen ion neutralization.

3. The oral care device (100) according to claim 1 or claim 2, comprising an actuator (112) configured to move in accordance with the electrical signal.

4. The oral care device (100) according to claim 3, wherein the control arrangement (102) is configured to generate an actuator control signal to control the movement of the actuator (112), the actuator control signal being in phase with the electrical signal, or a phase difference being defined between the actuator control signal and the electrical signal.

5. The oral care device (100) according to claim 3 or claim 4, comprising at least one cleaning element (114) for mechanically or fluidically cleaning inside the subject's oral cavity, said at least one cleaning element being arranged such that said movement of the actuator respectively causes movement of or fluid delivery from the at least one cleaning element; optionally wherein the at least one cleaning element (114) comprises bristles or a fluid-delivering nozzle for cleaning surfaces inside the subject's oral cavity.

6. The oral care device (100) according to any of claims 1 to 5, wherein said electrical signal comprises a periodic variation in at least one of amplitude, phase and frequency.

7. The oral care device (100) according to any of claims 1 to 6, wherein the electrical signal is a continuous waveform.

8. The oral care device (100) according to any of claims 1 to 6, wherein the electrical signal is a pulsed electrical signal.

9. The oral care device (100) according to claim 8, wherein a duty cycle of the pulsed electrical signal is less than or equal to 90%, preferably less than or equal to 50%.

10. The oral care device (100) according to any of claims 1 to 9, wherein the electrical signal comprises a bidirectional waveform supplied to the electrodes.

11. The oral care device (100) according to claim 10, wherein a positive peak amplitude (152) of the bidirectional waveform is different from a negative peak amplitude (154) of the bidirectional waveform; optionally wherein one of the positive and negative peak amplitudes is at least 1.1 times the other of the positive and negative peak amplitudes.

12. The oral care device (100) according to any of claims 1 to 11, wherein the control arrangement (102) is configured to provide the electrical signal to the pair of electrodes (104A, 104B) for a predetermined time period before terminating the provision of the electrical signal to the pair of electrodes.

13. The oral care device (100) according to any of claims 1 to 12, wherein the pair of electrodes (104A, 104B) are insertable into the subject's oral cavity, said aqueous solution comprising the subject's saliva and/or one or more oral care agents.

14. The oral care device (10) according to any of claims 1 to 13, comprising a cleaning unit (132) for inserting into the subject's oral cavity, said cleaning unit having a recess in which one or both of the pair of electrodes (104A, 104B) is or are mounted; and/or wherein the pair of electrodes are arranged with one of the electrodes defining an outer electrode which at least partially surrounds the other of the electrodes that defines an inner electrode and/or wherein the pair of electrodes are interdigitated.

15. A method of operating an oral care device comprising a pair of electrodes (104A, 104B) for contacting an aqueous solution, the method comprising providing an electrical signal to the pair of electrodes to cause generation of hydrogen ions from water in the aqueous solution to deliver within a subject's oral cavity, a variation of the electrical signal controlling said generation of hydrogen ions.
